# DEMANDE DE BREVET EUROPEEN

(11) **EP 4 062 931 A1**
(43) Date de publication de la demande: **28.09.2022**
(21) Numéro de dépôt: 21180477.8
(22) Date de dépôt: 18.06.2021
(51) Int. Cl.: A61K 39/215, A61K 9/00

(54) **NOUVELLE APPLICATION D'UNE COMPOSITION IMMUNOGENE OU VACCINALE**

(30) Priorité: 22.03.2021 DE 102021001467
(71) Demandeur: EYE VACC, 4031 Liège (Angleur) (BE)
(72) Inventeur: SCHRAGE, Norbert, D-52070 AIX-LA-CHAPELLE (DE); HOLZER, Frank, 66129 SAARBRÜCKEN (DE); BLOMET, Joël, 75015 PARIS (FR); DESMECHT, Daniel, B-4141 LOUVEIGNE (BE)
(74) Mandataire: Plasseraud IP

(57) **Abrégé**

La présente invention concerne une composition immunogène ou vaccinale comprenant un marqueur, pour son utilisation dans la prévention et/ou le traitement d'une infection causée par au moins un agent pathogène, caractérisée en ce qu'elle est administrée sur la muqueuse oculaire et/ou la muqueuse uro-génitale.

## Description

En plus du système immunitaire systémique, notre organisme contient un système immunitaire associé aux muqueuses (également dénommé MALT, Mucosa Associated Lymphoid Tissue). Les muqueuses contiennent localement leur propre système immunitaire, par exemple au niveau du tractus digestif (GALT, Gut Associated Lymphoid Tissue), au niveau du nez (NALT, Nasal Associated Lymphoid Tissue), ou encore au niveau de l'œil (CALT, Conjunctiva Associated Lymphoid Tissue

En effet, les sites d'entrée des agents pathogènes dans l'organisme se situent généralement au niveau des muqueuses de l'œil, du nez, de la bouche ou du tractus gastro-intestinal. Notre organisme contient ainsi un grand nombre de mécanismes de défense cellulaires et biochimiques, directement au niveau de ces muqueuses, qui s'activent au contact des agents pathogènes. Typiquement le système immunitaire des muqueuses comprend les cellules épithéliales, les cellules de l'immunité innée et les cellules dendritiques qui sont à l'interface entre l'immunité innée et l'immunité spécifique (acquise). Les muqueuses peuvent ainsi contenir des cellules de l'immunité innée, des cellules immunocompétentes, des cellules mémoires et des cellules productrices d'anticorps.

Dans le cas d'agents pathogènes colonisant les muqueuses et hautement contagieux, l'agent pathogène se multiplie si rapidement après avoir colonisé les muqueuses que l'infection des muqueuses et des couches plus profondes progresse plus vite que l'organisme ne peut établir une immunité efficace pour éliminer le pathogène. Ceci est typiquement le cas de l'infection actuelle par le coronavirus 2 du syndrome respiratoire aigu sévère (SRAS-CoV-2 ou Severe Acute Respiratory Syndrome CoronaVirus 2). En effet, la Covid-19 (ou maladie à coronavirus 2019) présente principalement une infection et une inflammation des muqueuses avec une maladie générale grave secondaire, comme cela est également le cas pour la grippe, l'herpès ou la poliomyélite.

Après avoir survécu à une primo-infection par un agent pathogène, l'organisme développe une immunité systémique (sérique) contre cet agent. Par ailleurs, si l'agent pathogène a été en contact avec les muqueuses de l'organisme, une immunité des muqueuses est également développée, en plus de l'immunité systémique.

Des vaccins permettant de développer l'immunité des muqueuses sont déjà connus. Ces derniers sont administrés essentiellement par voie nasale ou orale. Ils offrent l'avantage singulier d'induire une réponse immunitaire protectrice, tant au niveau des muqueuses qu'au niveau systémique. Ils visent également à empêcher le franchissement des muqueuses, porte d'entrée de la plupart des agents pathogènes bactériens et viraux. C'est par exemple le cas du vaccin contre la grippe FluMist^{®} qui est administré par vaporisation nasale, ou encore les vaccins contre la poliomyélite qui s'administrent par voie orale.

La vaccination contre un virus de la grippe (virus influenza A), par voie oculaire, chez des furets a également été étudiée dans l'article Eyedrop Vaccination Induced Systemic and Mucosal Immunity against Influenza Virus in Ferrets de Sangchul Yoon, Eun-Do Kim, Min-Suk Song, Soo Jung Han, Tae Kwann Park, Kyoung Sub Choi, Young-Ki Choi, et Kyoung Yul Seo; PLoS One. 2016; 11(6): e0157634. La vaccination par voie oculaire a également été étudiée chez des souris par l'équipe Kyoung Yul Seo, Soo Jung Han, Hye-Ran Cha, Sang-Uk Seo, Joo-Hye Song, So-Hyang Chung, et Mi-Na Kweon (Eye Mucosa: An Efficient Vaccine Delivery Route for Inducing Protective Immunity ; J Immunol 2010; 185:3610-3619), bien que les souris ne soient pas des hôtes naturels du virus. Cependant, à ce jour aucun vaccin oculaire n'a été autorisé.

Afin de vacciner la population mondiale contre diverses infections causées par au moins un agent pathogène, notamment des infections bactériennes ou virales, il existe toujours un besoin pour des vaccins de plus en plus efficaces.

Une vaccination efficace s'entend également d'un vaccin administré aux quantités recommandées, et/ou correctement administré au niveau du site d'administration. Des erreurs de vaccination ont en effet déjà été relevées, notamment en raison d'un mauvais site d'injection (par exemple l'administration sous-cutanée d'un vaccin BCG alors que celui-ci doit être administré par voie intradermique), ou en raison d'administration de doses de vaccins supérieures aux doses recommandées.

Cependant, à ce jour, il n'existe pas de moyen permettant de vérifier facilement la quantité de vaccin administrée et/ou la correcte application au niveau du site d'administration.

Il existe ainsi un besoin pour des vaccins efficaces, dont on peut facilement s'assurer de la bonne administration, notamment en termes de quantité administrée et/ou du site d'administration.

La présente invention répond à ce besoin grâce à un nouveau mode de vaccination contre des infections causées par au moins un agent pathogène qui permet (i) d'améliorer l'immunité (la double immunité -systémique et des muqueuses- assure en effet une meilleure protection vis-à-vis de l'agent pathogène, à la fois pour l'hôte vacciné et pour les autres car cela pourrait limiter les risques de transmission de l'agent pathogène), et (ii) de s'assurer de la bonne administration du vaccin, que ce soit en termes de quantité administrée et/ou du site d'administration.

La présente invention fournit ainsi un nouveau mode de vaccination contre des infections causées par au moins un agent pathogène, plus particulièrement les infections bactériennes et/ou virales, plus efficace que les autres modes de vaccination précédemment utilisés, et qui soit simple à mettre en œuvre.

La présente invention permet de développer une immunité des muqueuses contre au moins un agent pathogène, en ciblant la muqueuse oculaire et/ou la muqueuse uro-génitale.

La présente invention concerne ainsi une composition immunogène ou vaccinale comprenant un marqueur, pour son utilisation dans la prévention et/ou le traitement d'une infection causée par au moins un agent pathogène, caractérisée en ce qu'elle est administrée sur la muqueuse oculaire et/ou la muqueuse uro-génitale.

L'intérêt de l'administration d'une telle composition est d'établir à la fois une immunité des muqueuses et une immunité sérique contre au moins un agent pathogène. Cette double immunité permet de mieux protéger l'organisme vis-à-vis de l'agent pathogène et limite le risque d'infections, sans subir les graves effets secondaires systémiques qui peuvent être constatés avec certains vaccins (par exemple les risques de thromboses observés avec certains vaccins contre la Covid-19 de type vecteur viral non réplicatif administrés par voie intramusculaire). En effet, dans le cas de la présente invention, étant donné que l'immunité sérique est acquise indirectement *via* une immunisation des muqueuses, un accident thrombotique n'est donc pas attendu.

L'intérêt de la présence d'un marqueur dans ladite composition permet également de détecter et/ou vérifier la quantité administrée et/ou le site d'administration, ceci afin de s'assurer d'une application sûre et efficace de la composition immunogène ou vaccinale.

L'expression « composition immunogène » et/ou « composition vaccinale » selon l'invention s'entend d'une composition qui induit une réponse immunitaire contre au moins un agent pathogène après administration chez le sujet. Une composition vaccinale permet notamment de générer une immunité, plus particulièrement une réponse immunitaire protectrice et adaptative contre au moins un agent pathogène, plus particulièrement un virus et/ou une bactérie. Cette réponse immunitaire peut être humorale et/ou cellulaire.

Le terme « un marqueur » selon l'invention s'entend de toute substance mesurable ou indicatrice qui peut être administrée dans une composition immunogène ou vaccinale. Il s'agit donc d'un marqueur pharmaceutiquement acceptable. Selon un mode de réalisation particulier, le marqueur permet de quantifier et/ou de visualiser l'application de ladite composition immunogène ou vaccinale sur la muqueuse oculaire et/ou la muqueuse uro-génitale. Plus précisément, la quantification s'entend de la mesure de la quantité de composition immunogène ou vaccinale administrée. Selon un mode de réalisation, le marqueur est une substance indicatrice tel qu'un colorant. A titre d'exemple les colorants de type fluorescéine ou indocyanine peuvent être utilisés.

L'expression « une infection causée par au moins un agent pathogène » selon l'invention s'entend d'une maladie causée par un ou plusieurs agents infectieux.

Le terme « agent pathogène » selon l'invention s'entend de tout agent infectieux capable de provoquer une maladie chez son hôte. Plus particulièrement, un agent pathogène s'entend ainsi d'un virus, d'une bactérie, d'un parasite, d'un champignon et/ou d'un prion.

L'expression « virus, bactérie, parasite, champignon et/ou prion » selon l'invention s'entend d'un virus, d'une bactérie, d'un parasite, d'un champignon et/ou d'un prion qui sont considérés ou peuvent être considérés comme agent pathogène pour un organisme, de préférence un organisme humain. Typiquement cela ne s'entend donc pas de bactéries bénéfiques à l'organisme telles que les probiotiques. Plus précisément l'expression « virus, bactérie, parasite, champignon ou prion » s'entend donc d'un virus, d'une bactérie, d'un parasite, d'un champignon et/ou d'un prion pathogène.

L'expression « infection causée par au moins un agent pathogène » s'entend d'une infection causée par une bactérie et/ou un virus et/ou un parasite et/ou un champignon et/ou un prion pathogène. Selon un mode de réalisation particulier, l'infection est une infection bactérienne et/ou virale, et plus particulièrement, l'infection est soit bactérienne, soit virale. A titre d'exemple, l'infection est choisie parmi : la grippe, l'herpès, la mononucléose infectieuse, la poliomyélite, la salmonellose, la fièvre typhoïde ou paratyphoïde, la tuberculose, le choléra, la maladie de Lyme, d'une infection causée par *Staphylococcus aureus* (notamment *Staphylococcus aureus* résistant à la méticilline), une infection causée par pneumocoque, une infection causée par méningocoque, la fièvre jaune, les oreillons, une gastroentérite à rotavirus, la rougeole, la rubéole, la varicelle, le zona, les hépatites virales, l'encéphalite japonaise, la coqueluche, la diphtérie, le tétanos, un infection causée par un coronavirus telle que le SARS ou la Covid-19, une infection par un virus du papillome humain, une maladie à prions telle que la maladie de Creutzfeldt-Jakob, une infection causée par un amibe telle qu'une amibiase, la syphilis, une mycose, le paludisme, ou une infection causée par bactéries GNMR (bactéries à Gram-Negatif Multi-résistantes).

L'expression « la prévention d'une infection » selon l'invention s'entend de la prophylaxie. L'administration de la composition immunogène ou vaccinale selon l'invention permet notamment d'empêcher ou de réduire le risque de développer ladite infection, et/ou réduit le cas échéant le risque de développer des formes dites sévères de la maladie (c'est-à-dire avec des symptômes graves). L'administration de la composition immunogène ou vaccinale selon l'invention pourrait également empêcher ou diminuer le risque de transmission de l'agent pathogène, plus particulièrement du virus ou de la bactérie, typiquement d'une personne à une autre. Selon un mode de réalisation particulier, la composition immunogène ou vaccinale pour la prévention d'une infection selon l'invention est administrée chez un sujet n'étant pas contaminé, de préférence n'étant pas infecté, par l'agent pathogène, de préférence le virus et/ou la bactérie.

L'expression « le traitement d'une infection » selon l'invention s'entend de la thérapie. L'administration de la composition immunogène ou vaccinale selon l'invention peut en effet être envisagée, afin de stimuler les défenses naturelles de l'organisme, alors même que la personne est a *minima* déjà contaminée par l'agent pathogène, de préférence le virus et/ou la bactérie. Selon un mode de réalisation particulier, la composition immunogène ou vaccinale pour le traitement d'une infection selon l'invention est administrée chez un sujet étant contaminé, et/ou étant infecté, par l'agent pathogène, de préférence le virus et/ou la bactérie.

De préférence, l'invention concerne ainsi une composition immunogène ou vaccinale comprenant un marqueur, pour son utilisation dans la prévention d'une infection causée par au moins un agent pathogène, notamment une infection bactérienne et/ou virale, caractérisée en ce qu'elle est administrée sur la muqueuse oculaire et/ou la muqueuse uro-génitale. Selon un mode de réalisation, l'invention concerne une composition immunogène ou vaccinale comprenant un marqueur, pour son utilisation dans la prévention d'une infection bactérienne, caractérisée en ce qu'elle est administrée sur la muqueuse oculaire et/ou la muqueuse uro-génitale. Selon un autre mode de réalisation, l'invention concerne une composition immunogène ou vaccinale comprenant un marqueur, pour son utilisation dans la prévention d'une infection virale, caractérisée en ce qu'elle est administrée sur la muqueuse oculaire et/ou la muqueuse uro-génitale.

L'expression « la muqueuse oculaire » selon l'invention s'entend de la conjonctive et la cornée. La conjonctive est la membrane muqueuse transparente qui recouvre la face interne des paupières supérieures et inférieures et qui couvre la surface antérieure du globe oculaire. Plus précisément, la muqueuse oculaire s'entend donc à la fois de la conjonctive tarsale et de la conjonctive bulbaire, de même que les culs-de-sac conjonctivaux.

L'expression « la muqueuse uro-génitale » selon l'invention s'entend des muqueuses de l'appareil urinaire et/ou génital, tant l'appareil masculin que l'appareil féminin. Plus précisément, la muqueuse uro-génitale s'entend donc du tractus uro-génital.

Le ciblage de la muqueuse oculaire et/ou la muqueuse uro-génitale permet de cibler des muqueuses qui (i) ne participent en elles-mêmes que dans une faible mesure à la diffusion de l'agent pathogène, mais (ii) qui présentent en même temps une immunocompétence très élevée, qui permet d'immuniser très rapidement l'organisme, notamment avant une infection des voies respiratoires. Ce mode de réalisation permet par ailleurs de diminuer les éventuels risques d'effet secondaires graves liés à la vaccination.

Une des hypothèses, non limitative, des inventeurs est en effet qu'une infection de l'œil par un agent pathogène entraîne la formation rapide d'une immunité, permettant à l'organisme de gagner du temps, de façon à ce que lorsque la contamination et/ou l'infection progresse dans l'organisme, notamment via le nez, l'organisme est déjà immunisé. Cette progression de la contamination et/ou l'infection de l'œil vers le nez pourrait s'expliquer grâce à la présence du canal nasolacrimal qui relie le nez aux yeux. Une infection par le canal nasolacrimal a déjà été décrite dans le cas de kératite causée par des virus (de type *keratitis epidemica*). L'évolution typique de cette maladie commence par une infection de la conjonctive par des gouttelettes et se manifeste par une conjonctivite sévère suivie d'une pharyngite. Cela pourrait également expliquer pourquoi des sujets, notamment du personnel médical, ont développé une immunité systémique contre le SRAS-CoV-2 après avoir développé une conjonctivite positive au SRAS-CoV-2.

De préférence, ladite composition immunogène ou vaccinale est administrée sur la muqueuse oculaire. Selon un mode de réalisation, l'invention concerne ainsi une composition immunogène ou vaccinale comprenant un marqueur, pour son utilisation dans la prévention et/ou le traitement d'une infection bactérienne, caractérisée en ce qu'elle est administrée sur la muqueuse oculaire. Selon un autre mode de réalisation, l'invention concerne ainsi une composition immunogène ou vaccinale comprenant un marqueur, pour son utilisation dans la prévention et/ou le traitement d'une infection virale, caractérisée en ce qu'elle est administrée sur la muqueuse oculaire.

Selon un mode de réalisation encore plus préféré, la présente invention concerne une composition immunogène ou vaccinale comprenant un marqueur, pour son utilisation dans la prévention d'une infection bactérienne, caractérisée en ce qu'elle est administrée sur la muqueuse oculaire. Selon un autre mode de réalisation encore plus préféré, l'invention concerne ainsi une composition immunogène ou vaccinale comprenant un marqueur, pour son utilisation dans la prévention d'une infection virale, caractérisée en ce qu'elle est administrée sur la muqueuse oculaire.

Selon l'invention, ladite composition immunogène ou vaccinale peut être administrée sur un seul ou sur les deux yeux. Contrairement à une administration intramusculaire, sous-cutanée ou intradermique d'un vaccin qui nécessite une organisation et un effort médical accru (e.g. préparation des seringues, nécessité de disposer d'un équipement de réanimation en cas de réaction grave à la vaccination, ...), l'injection sur une muqueuse oculaire, typiquement par collyre, est bien plus simple et rapide à mettre en œuvre. En effet, les risques graves sont diminués (par exemple les risques de thrombose comme indiqués ci-dessus), de même que les graves risques allergiques qui se manifestent principalement par des picotements aux yeux, un larmoiement ou encore des yeux rouges. La vaccination par collyre permet par ailleurs de vacciner bien plus rapidement et à plus grande échelle.

Selon un mode de réalisation, ladite composition immunogène ou vaccinale comprend une ou plusieurs substances permettant de provoquer une réaction immunitaire contre un ou plusieurs agents pathogènes, notamment un ou plusieurs virus et/ou une ou plusieurs bactéries. Typiquement, ladite composition immunogène ou vaccinale selon l'invention, comprend un ou plusieurs antigènes dudit agent pathogène, notamment dudit virus ou de ladite bactérie. De préférence, le ou lesdits antigène(s) sont spécifiques de l'agent pathogène responsable de l'infection, notamment du virus ou de la bactérie responsable de l'infection.

Selon un mode de réalisation, la composition immunitaire ou vaccinale permet de provoquer une réponse immunitaire contre au moins un agent pathogène (notamment un virus et/ou une bactérie), dès lors qu'elle comprend au moins un antigène dudit agent pathogène, un micro-organisme (par exemple un virus ou une bactérie) qui peut produire au moins un antigène dudit agent pathogène, ou bien qu'elle comprend le matériel génétique nécessaire permettant l'expression d'au moins un antigène dudit agent pathogène (typiquement un ARNm). Dans le premier cas, l'antigène sera en contact avec la muqueuse directement dès l'administration et dans les second et troisième cas, une période de latence pourra être attendue, le temps que l'antigène soit produit après administration de la composition. De préférence, ledit micro-organisme n'est pas pathogène *per se*, la seule réaction immunitaire qu'il peut provoquer est celle liée à l'antigène dudit agent pathogène.

Selon un mode de réalisation particulier, les substances/antigènes permettant de provoquer une réaction immunitaire contre ledit agent pathogène sont choisis parmi : (i) un virus inactivé, (ii) une bactérie ou toxine bactérienne inactivée, (iii) un virus atténué, (iv) une bactérie ou toxine bactérienne atténuée, (v) un parasite atténué ou inactivé, (vi) un champignon ou toxine fongique atténué(e) ou inactivé(e), (vii) un prion, (viii) un micro-organisme génétiquement modifié (exprimant ou pouvant exprimer au moins un antigène d'un agent pathogène, optionnellement inactivé ou atténué), (ix) un acide ribonucléique messager (ARNm) codant pour un ou plusieurs antigènes d'un virus ou d'une bactérie ou d'un parasite ou d'un champignon (tels qu'une protéine virale ou bactérienne), (x) un acide désoxyribonucléique (ADN) codant pour un ou plusieurs antigènes d'un virus ou d'une bactérie ou d'un parasite ou d'un champignon (tels qu'une protéine virale ou bactérienne), (xi) une protéine virale ou bactérienne ou parasitaire ou fongique ou un ou plusieurs fragments de celle-ci, ou (xii) une protéine de fusion comprenant une protéine virale et/ou bactérienne et/ou parasitaire et/ou fongique et/ou un prion, ou un ou plusieurs fragments de celle(s)-ci. Un fragment d'une protéine virale et/ou bactérienne et/ou parasitaire et/ou fongique et/ou prion contient de préférence le ou les épitopes immunodominants ou biosimilaires de celle-ci. Selon un mode de réalisation, le fragment de ladite protéine est un fragment immunogène.

Selon un mode de réalisation, un micro-organisme génétiquement modifié s'entend d'un micro-organisme (de préférence inactivé ou atténué) exprimant ou pouvant exprimer un ou plusieurs antigènes d'un virus ou d'une bactérie ou d'un parasite ou d'un champignon (tels qu'une protéine virale ou bactérienne), ou d'un micro-organisme (notamment virus, bactérie ou parasite) génétiquement modifié pour les rendre incapable d'infecter un organisme hôte (par exemple en les empêchant de pénétrer une cellule cible ou en les empêchant de se multiplier).

Selon un mode de réalisation particulier, les substances/antigènes permettant de provoquer une réaction immunitaire contre ledit virus et/ou ladite bactérie sont choisis parmi : (i) un virus inactivé, (ii) une bactérie ou toxine bactérienne inactivée, (iii) un virus atténué, (iv) une bactérie ou toxine bactérienne atténuée, (v) un micro-organisme génétiquement modifié (de préférence inactivé ou atténué) exprimant ou pouvant exprimer un ou plusieurs antigènes d'un virus ou d'une bactérie (tels qu'une protéine virale ou bactérienne), (vi) un acide ribonucléique messager (ARNm) codant pour un ou plusieurs antigènes d'un virus ou d'une bactérie (tels qu'une protéine virale ou bactérienne), (vii) un acide désoxyribonucléique (ADN) codant pour un ou plusieurs antigènes d'un virus ou d'une bactérie (tels qu'une protéine virale ou bactérienne), (viii) une protéine virale ou bactérienne ou un ou plusieurs fragments de celle-ci, ou (ix) une protéine de fusion comprenant une protéine virale et/ou bactérienne ou un ou plusieurs fragments de celle(s)-ci.

La composition selon l'invention peut comprendre une ou plusieurs substances (antigènes) permettant de provoquer une réaction immunitaire contre un ou plusieurs agents pathogènes, notamment contre un ou plusieurs virus, contre une ou plusieurs bactéries ou contre un ou plusieurs virus et une ou plusieurs bactéries. Selon un mode de réalisation, les différentes substances (antigènes) peuvent cibler différentes parties du même virus ou de la même bactérie ou du même champignon ou du même parasite (par exemple différents épitopes sur des protéines virales ou bactériennes), des souches différentes du même virus ou de la même bactérie ou du même champignon, des variants du même virus ou de la même bactérie ou du même champignon ou du même parasite, voire plusieurs virus différents et/ou plusieurs bactéries différentes et/ou champignons différents et/ou parasites différents (vaccin combiné). La composition immunogène ou vaccinale selon l'invention peut ainsi être monovalente ou polyvalente. Une composition immunogène ou vaccinale monovalente protège contre un seul agent pathogène, alors que la composition immunogène ou vaccinale polyvalente protège contre plusieurs agents pathogènes.

Selon un mode de réalisation, l'expression « une protéine virale ou bactérienne ou fongique ou parasitaire » s'entend plus particulièrement des protéines structurales et des protéines accessoires du virus, ou des protéines impliquées dans l'adhérence, l'adhésion, l'invasion et/ou l'internalisation de la bactérie dans une cellule de l'organisme, ou des protéines impliquées dans l'adhérence, l'adhésion et/ou l'invasion du champignon dans un tissu, ou des protéines impliquées dans l'adhérence, l'adhésion, l'invasion et/ou l'internalisation du parasite dans une cellule de l'organisme (tel qu'un globule rouge), ou encore le cas échéant des protéines sécrétées par le virus, la bactérie, le champignon ou le parasite. De préférence, ladite protéine virale protéine bactérienne, protéine fongique ou protéine parasitaire est choisie parmi : une protéine d'enveloppe, une protéine de matrice, une protéine membranaire ou transmembranaire, une protéine de surface ou une anatoxine. A titre d'exemple, une protéine de surface bactérienne ou fongique peut être une adhésine et une protéine d'enveloppe virale peut être une protéine de spicule.

Selon l'invention, les protéines virales s'entendent des protéines natives du virus (les protéines du virus telles que retrouvées dans la nature), des protéines mutées ou variantes par rapport aux protéines natives ou des protéines synthétisées (modifiées, mutées ou non).

Selon l'invention, les protéines bactériennes s'entendent des protéines natives de la bactérie (les protéines de la bactérie telles que retrouvées dans la nature), des protéines mutées ou variantes par rapport aux protéines natives ou des protéines synthétisées (modifiées, mutées ou non).

Selon l'invention, les protéines fongiques s'entendent des protéines natives du champignon (les protéines du champignon telles que retrouvées dans la nature), des protéines mutées ou variantes par rapport aux protéines natives ou des protéines synthétisées (modifiées, mutées ou non).

Selon l'invention, les protéines parasitaires s'entendent des protéines natives du parasite (les protéines du parasite telles que retrouvées dans la nature), des protéines mutées ou variantes par rapport aux protéines natives ou des protéines synthétisées (modifiées, mutées ou non).

A titre d'exemple, les agents pathogènes responsables des infections ciblées par la présente invention sont le virus de la grippe, le virus de l'herpès simplex de type 1 ou 2 , le virus d'Epstein-Barr, le poliovirus, la bactérie du genre *Salmonella* (de préférence *Salmonella enterica- Typhi ou Paratyphi A*, *B*, *C*), la bactérie du genre *Mycobacterium* (de préférence *Mycobacterium tuberculosis*), la bactérie *Vibrio choleræ*, une bactérie appartenant au genre *Staphylococcus* (de préférence *Staphylococcus aureus*), un pneumocoque, un méningocoque, le virus de la fièvre jaune, le virus ourlien, le rotavirus, le virus de la rougeole, le virus de la rubéole, le virus de la varicelle ou varicellezona, les virus dits A, B, C, D et E responsables des hépatites, le virus de l'encéphalite japonaise, les bactéries du genre *Bordetella* (de préférence *Bordetella pertussis* et *Bordetella parapertussis*), la bactérie du genre *Corynebacterium* (de préférence *C. diphtheriae*, C. *ulcerans*, *C. pseudotuberculosis*), la bactérie *Clostridium tetani*, un coronavirus tel que le SARS-CoV ou le SARS-CoV-2, ou le virus du papillome humain, une bactérie de la famille des spirochètes (notamment *Borrelia burgdorferi ou Treponema pallidum).*

La composition immunogène ou vaccinale selon l'invention est administrée chez un sujet. Selon un mode de réalisation, ladite composition immunogène ou vaccinale est utilisée dans la prévention et/ou le traitement d'une infection causée par au moins un agent pathogène chez un humain. Selon l'invention, un humain peut être un nourrisson, un enfant ou un adulte.

Selon un autre mode de réalisation, les applications vétérinaires de ladite composition immunogène ou vaccinale selon l'invention sont envisagées. Dans un tel cas, le sujet est ainsi un animal.

Selon un mode de réalisation, ladite composition immunogène ou vaccinale est administrée à un sujet en une quantité immunologiquement efficace. Une telle quantité peut être déterminée par le praticien. L'expression « une quantité immunologiquement efficace » s'entend d'une quantité suffisante pour être efficace sur le plan préventif et/ou thérapeutique, notamment chez un sujet ayant besoin d'une telle prévention ou d'un tel traitement.

Selon un mode de réalisation, ledit marqueur est présent dans la composition immunogène ou vaccinale en une quantité suffisante à sa détection/visualisation.

Selon un mode de réalisation, ladite composition immunogène ou vaccinale est administrée à un sujet n'ayant jamais été contaminé, voire infecté, par l'agent pathogène, notamment le virus et/ou la bactérie, responsable de l'infection ou bien à un sujet ayant déjà été contaminé, voire infecté, par l'agent pathogène, notamment le virus et/ou la bactérie, responsable de l'infection (que la personne ait développé des symptômes (légers ou graves) ou ait été asymptomatique).

Selon un mode de réalisation, ladite composition immunogène ou vaccinale est administrée une fois ou plusieurs fois, de préférence une, deux ou trois fois, sur la muqueuse oculaire et/ou la muqueuse uro-génitale. Selon un mode de réalisation particulier, ladite composition immunogène ou vaccinale est administrée au moins deux fois sur la muqueuse oculaire et/ou la muqueuse uro-génitale (en d'autres termes deux doses de composition immunogène ou vaccinale sont administrées). Selon un mode de réalisation particulier, lorsque la composition immunogène ou vaccinale est administrée plusieurs fois, c'est la même muqueuse qui est ciblée : par exemple deux ou trois administrations sur la muqueuse oculaire ou deux ou trois administrations sur la muqueuse uro-génitale. La durée entre la première et la ou les autres administrations ultérieures est variable selon l'agent pathogène ciblé, par exemple quelques semaines pour une composition immunogène contre le virus Sars-CoV-2 ou quelques mois pour une composition immunogène contre le virus responsable de l'hépatite A.

Selon un mode de réalisation, ladite composition immunogène ou vaccinale est administrée sous forme de goutte ou sous forme de lyophilisat. Typiquement, lorsque l'administration est sous forme de goutte, cela s'entend que ladite composition immunogène ou vaccinale est une formulation liquide, et lorsque l'administration est sous forme de lyophilisat, ladite composition immunogène ou vaccinale est sous forme de poudre. Ladite poudre peut être appliquée directement au niveau des muqueuses, ou bien être appliquée sur un papier filtre, un polymère, un gel ou toute autre substance ou support approprié qui sera ensuite mis en contact avec la muqueuse oculaire et/ou la muqueuse uro-génitale. La composition immunogène ou vaccinale sera ainsi transférée du papier filtre, du polymère, du gel ou toute autre substance ou support approprié vers la muqueuse oculaire et/ou uro-génitale.

Selon un mode de réalisation, ladite composition immunogène ou vaccinale est administrée à l'aide d'un applicateur. Un applicateur permet notamment de faciliter l'administration de la composition, par exemple il facilite l'instillation des gouttes (de type aide-verseur) ou de la poudre, du lyophilisat. L'applicateur peut également être une ampoule, une seringue, un papier filtre (avec par exemple un fluide ou un lyophilisat), un flacon de goutte, un applicateur de dose unique, un applicateur de type spirale ou éponge vaginale, ...

Selon un mode de réalisation, la composition immunogène selon l'invention comprend ou est constituée d'une ou plusieurs substances (antigènes) permettant de provoquer une réaction immunitaire contre un agent pathogène, notamment un virus et/ou une bactérie. Selon un mode de réalisation, il peut être considéré que la composition immunogène selon l'invention est comprise dans une composition vaccinale (un vaccin). A titre d'exemple, la composition immunogène ou vaccinale selon l'invention peut être le vaccin à ARNm Pfizer/BioNTech, généralement dénommé Bnt162b2 ou Comirnaty^{®} ou le vaccin à vecteur viral non réplicatif d'AstraZeneca, généralement dénommé Vaxzevria^{®}, ChAdOx1-S ou COVID-19 Vaccine AstraZeneca lorsque le virus est le SARS-CoV-2, la composition immunogène ou vaccinale selon l'invention peut être le vaccin Dukoral^{®} lorsque la bactérie est *Vibrio choleræ*, ou encore le vaccin Revavix^{®} (vaccin combiné contre la bactérie *Corynebacterium diphtheriae* responsable de la diphtérie, contre la bactérie *Clostridium tetani* responsable du tétanos et contre un poliovirus responsable de la poliomyélite).

Selon un mode de réalisation préféré, ladite composition vaccinale comprend un adjuvant et/ou un excipient.

Selon un mode de réalisation, ladite composition immunogène ou vaccinale comprend un véhicule pharmaceutiquement acceptable.

L'adjuvant et/ou l'excipient et/ou le diluant et/ou le véhicule pharmaceutiquement acceptable sont ceux classiquement utilisés. Par exemple, le véhicule pharmaceutiquement acceptable peut être un solvant ou une solution permettant de diluer la composition avant administration par voie oculaire ou sur la muqueuse uro-génitale.

Selon une mode de réalisation, une composition immunogène ou vaccinale selon l'invention, peut en outre comprendre une ou plusieurs substances additionnelles. De préférence, ladite substance additionnelle est choisie parmi :
- un conservateur,
- un tensioactif,
- un additif.

A titre d'exemple, un conservateur peut être un conservateur antimicrobien qui vise à empêcher la contamination microbienne de la composition immunogène ou vaccinale. Selon l'invention, le conservateur est un conservateur compatible avec les muqueuses.

Afin d'obtenir une antigénicité élevée et donc une réponse immunologique élevée, des substances, par exemple des tensioactifs, peuvent être ajoutées à la composition immunogène ou vaccinale, afin de prolonger le temps d'exposition sur la muqueuse oculaire et/ou uro-génitale, et éventuellement permettre de calculer le temps d'exposition de ladite composition sur la muqueuse.

D'autres substances, telles que des additifs, peuvent être ajoutées pour conférer des propriétés avantageuses à la composition immunogène ou vaccinale, par exemple une substance améliorant la pénétration de la composition immunogène ou vaccinale dans la muqueuse ou une substance permettant de prolonger le temps de contact entre la muqueuse et la composition (par exemple l'acide hyaluronique, un ou plusieurs polymères pour former un hydrogel (e.g. des carbomères), des dérivés de cellulose...).

La présente invention concerne également une méthode de prévention et/ou de traitement d'une infection causée par au moins un agent pathogène chez un sujet comprenant l'administration d'une composition immunogène ou vaccinale sur la muqueuse oculaire et/ou la muqueuse uro-génitale. Plus particulièrement, la présente invention concerne une méthode pour induire une réponse immunitaire protectrice contre au moins un agent pathogène, notamment un virus et/ou une bactérie, comprenant l'administration d'une composition immunogène ou vaccinale sur la muqueuse oculaire et/ou la muqueuse uro-génitale.

La présente invention concerne également l'utilisation d'une composition immunogène ou vaccinale contre au moins un agent pathogène, notamment un virus et/ou une bactérie, pour la préparation d'un médicament destiné à la prévention et/ou au traitement d'une infection causée par au moins un agent pathogène, et ledit médicament étant destiné à être administré sur la muqueuse oculaire et/ou la muqueuse uro-génitale.

### FIGURES

La **Figure 1** représente les résultats du groupe 1 de hamsters.
La **Figure 2** représente les résultats du groupe 2 de hamsters.

### EXEMPLES

### Exemple 1 : Exemples de compositions liquides

Le Tableau 1 ci-après, synthétise des compositions selon l'invention qui peuvent être utilisées.

**Tableau 1 : Compositions liquides**

| | **Antigène ou vaccin utilisé** | **Dose virale/concentration** | **Nombre et type d'applications** |
|---|---|---|---|
| **Composition 1** | Le vaccin à vecteur viral non réplicatif d'AstraZeneca, généralement dénommé Vaxzevria^{®}, ChAdOx1-S ou COVID-19 Vaccine AstraZeneca, avec ajout d'un marqueur de type colorant | 0,1*10² -10*10⁸ virus par ml | - Une ou plusieurs applications, de préférence trois |
| | | Par exemple, des doses de 0,1 mL peuvent être administrées | |
| | | | - Application sur un ou les deux yeux |
| **Composition 2** | Le vaccin à vecteur viral non réplicatif de Gamaleya, généralement dénommé Spoutnik V ou Gam-COVID-Vac, avec ajout d'un marqueur de type colorant | 0,1*10² -10*10⁸ virus par ml | - Une ou plusieurs applications, de préférence trois |
| | | Par exemple, des doses de 0,1 mL peuvent être administrées | |
| | | | - Application sur un ou les deux yeux |
| **Composition 3** | Protéine virale du SARS-CoV-2 (de type protéine S), avec ajout d'un marqueur de type colorant | 1:10⁻¹²g/ml à 1g/ml | - Une ou plusieurs applications, de préférence trois |
| | | Par exemple, des doses de 0,1 mL peuvent être administrées | |
| | | | - Application sur un ou les deux yeux |
| **Composition 4** | Bactérie non pathogène génétiquement modifié pour produire une protéine virale du SARS-CoV-2, avec ajout d'un marqueur de type colorant | / | - Une ou plusieurs applications, de préférence trois |
| | | | - Application sur un ou les deux yeux |

### Exemple 2 : Exemples de compositions lyophilisées

Le Tableau 2 ci-après, synthétise des compositions selon l'invention qui peuvent être utilisées.

**Tableau 2 : Compositions lyophilisées**

| | **Antigène ou vaccin utilisé** | **Nombre et type d'applications** |
|---|---|---|
| **Composition 5** | Protéine virale lyophylisée, par exemple la protéine S, avec ajout d'un marqueur de type colorant | - Une ou plusieurs applications, de préférence trois |
| | | - Application sur un ou les deux yeux |
| **Composition 6** | Virus lyophilisée (SARS-Cov-2, virus inactivé ou virus atténué), avec ajout d'un marqueur de type colorant | - Une ou plusieurs applications, de préférence trois |
| | | - Application sur un ou les deux yeux |

### Exemple 3 : Immunisation de hamsters contre le SARS-Cov-2 par voie oculaire

Les hamsters possèdent le récepteur ACE2. Ils peuvent ainsi être contaminés par le SRAS-CoV-2 et tomber malades.

### 1. Comparaison de deux groupes de hamsters après deux injections de virus

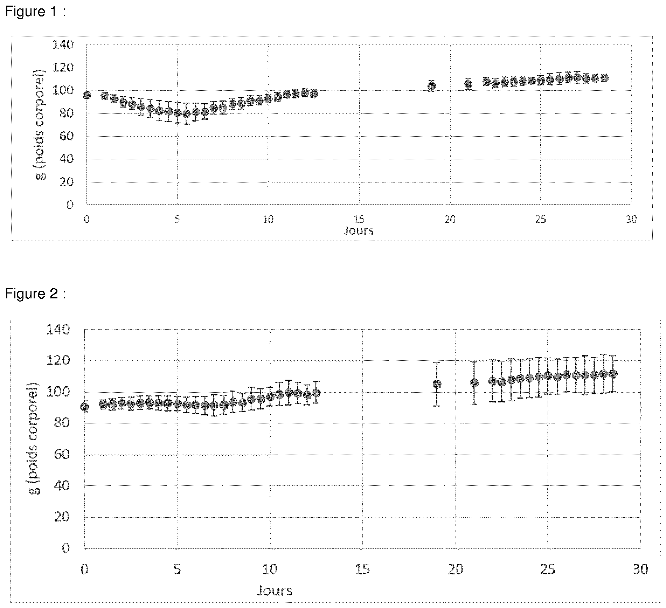

Deux groupes de hamster ont été étudiés.
Dans le groupe 1, n=7 hamsters ont été contaminés au SRAS-CoV-2 par injection nasale de 3x10⁶ d'un virus SARS-CoV-2 (souche B.1.214) et dans le groupe 2, n=7 hamsters ont été contaminés à l'aide d'un collyre contenant 3x10⁶ virus SARS-CoV-2 (souche B.1.214). Un marqueur de type colorant peut être ajouté au collyre.
Pendant l'observation, les hamsters du groupe 1 sont tombés malades, reconnaissables à une perte de poids significative, tandis que les hamsters du groupe 2 ne sont pas tombés malades.
En effet, on peut observer sur la Figure 1 que les hamsters du groupe 1 ont commencé à perdre du poids à compter du jour 2 avec un maximum au jour 5 et une normalisation au jour 12, ce qui indique que les hamsters sont tombés malades suite à la contamination/infection au SRAS-CoV-2 par injection nasale. Au contraire, on peut observer sur la Figure 2 que les hamsters du groupe 2 ne perdent pas de poids après inoculation du SARS-CoV-2 par voie oculaire. Ceci indique donc que les animaux restent en bonne santé.

Après 14 jours, les deux groupes ont été à nouveau exposés à une injection nasale contenant une dose virale de 3x10⁶ de SARS-CoV-2.
Dans les deux groupes les hamsters continuent leur prise de poids (voir les Figures 1 et 2). Plus particulièrement, au jour 21, les animaux du groupe 1 ne sont pas retombés malades après l'inhalation de la deuxième dose virale de SRAS-CoV-2. Cela signifie qu'ils sont devenus immunisés en raison de la maladie développée pendant les 10 premiers jours de l'expérience.
Egalement, au jour 21, les hamsters du groupe 2 ne sont pas tombés malades après l'inhalation de la deuxième dose virale de SRAS-CoV-2. Cela signifie que les hamsters sont devenus immunisés grâce à la première application par voie oculaire du SRAS-CoV-2.
Les hamsters du groupe 2 ont ainsi acquis une immunité avec l'application épi-oculaire, qui, contrairement au groupe 1, a été acquise sans développer une maladie générale grave.

### 2. Etude des hamsters après une injection de virus par voie oculaire

Par ailleurs, l'étude de n=10 autres hamsters qui ont reçu une seule application par voie oculaire d'une composition contenant une dose virale de 3x10⁶ SARS-CoV-2 (éventuellement en présence d'un marqueur de type colorant) ont montré qu'ils ont développé une forte production d'anticorps neutralisants vis-à-vis du virus (d'au moins 1:640 dans le sérum). Ces résultats signifient que par l'application épi-oculaire du virus, les hamsters ne développent pas de maladie, et ont acquis une immunité contre le SARS-CoV-2 qui est détectable dans le sérum.

### Exemple 4 : Etude de la propagation du SARS-Cov-2 lors de l'infection de la cavité nasale

Les Inventeurs ont analysé la propagation du virus chez des hamsters qui ont été contaminés au SARS-Cov-2 via une injection nasale, tels que décrit dans l'Exemple 3.

Les Inventeurs ont ainsi constaté que dans les premières 48 heures suivant l'entrée du virus dans le nez, l'infection progresse lentement d'une infection de la cavité nasale seule aux muqueuses, avec formation d'aérosols. Ces aérosols peuvent ensuite infecter les bronches et les alvéoles, notamment lors de l'inspiration, et contaminer d'autres personnes lors d'expiration.

## Revendications

1. Composition immunogène ou vaccinale comprenant un marqueur, pour son utilisation dans la prévention et/ou le traitement d'une infection causée par au moins un agent pathogène, **caractérisée en ce qu'**elle est administrée sur la muqueuse oculaire et/ou la muqueuse uro-génitale.

2. Composition immunogène ou vaccinale selon la revendication 1, **caractérisée en ce qu'**elle est administrée sur la muqueuse oculaire.

3. Composition immunogène ou vaccinale selon l'une quelconque des revendications précédentes, **caractérisée en ce que** le marqueur permet de quantifier et/ou de visualiser l'application de ladite composition immunogène ou vaccinale sur la muqueuse oculaire et/ou la muqueuse uro-génitale.

4. Composition immunogène ou vaccinale selon l'une quelconque des revendications précédentes, **caractérisée en ce que** le marqueur est un colorant.

5. Composition immunogène ou vaccinale selon l'une quelconque des revendications précédentes, **caractérisée en ce que** l'infection est une infection bactérienne et/ou virale.

6. Composition immunogène ou vaccinale selon l'une quelconque des revendications précédentes, **caractérisée en ce que** la composition comprend une ou plusieurs substances permettant de provoquer une réaction immunitaire contre une ou plusieurs agents pathogènes.

7. Composition immunogène ou vaccinale selon l'une quelconque des revendications précédentes, **caractérisée en ce que** l'agent pathogène est choisi parmi : un virus, une bactérie, un parasite, un champignon et/ou un prion, notamment un virus et/ou une ou plusieurs bactéries.

8. Composition immunogène ou vaccinale selon la revendication 6, **caractérisée en ce que** la substance permettant de provoquer une réaction immunitaire est choisi parmi : un virus inactivé, une bactérie ou toxine bactérienne inactivée, un virus atténué, une bactérie ou toxine bactérienne atténuée, un parasite atténué ou inactivé, un champignon ou toxine fongique atténué(e) ou inactivé(e), un prion, un micro-organisme génétiquement modifié, d'une bactérie, d'un parasite ou d'un champignon, un acide ribonucléique messager (ARNm) codant pour un ou plusieurs antigènes d'un virus, d'une bactérie, d'un parasite ou d'un champignon, un acide désoxyribonucléique (ADN) codant pour un ou plusieurs antigènes d'un virus, d'une bactérie, d'un parasite ou d'un champignon, ou une protéine virale ou bactérienne ou parasitaire ou fongique ou un ou plusieurs fragments de celle-ci, une protéine de fusion comprenant une protéine virale et/ou bactérienne et/ou parasitaire et/ou fongique et/ou un prion, ou un ou plusieurs fragments de celle(s)-ci.

9. Composition immunogène ou vaccinale selon la revendication 8, dans laquelle ladite protéine virale ou bactérienne ou parasitaire ou fongique est choisie parmi : une protéine d'enveloppe, une protéine de matrice, une protéine membranaire ou transmembranaire, une protéine de surface ou une anatoxine.

10. Composition immunogène ou vaccinale selon l'une quelconque des revendications précédentes, **caractérisée en ce qu'**elle est administrée sous forme de goutte ou sous forme de lyophilisat.

11. Composition immunogène ou vaccinale selon l'une quelconque des revendications précédentes, **caractérisée en ce qu'**elle est administrée à l'aide d'un applicateur.

12. Composition immunogène ou vaccinale selon l'une quelconque des revendications précédentes, pour son utilisation dans la prévention et/ou le traitement d'une infection causée par au moins un agent pathogène choisie parmi : la grippe, l'herpès, la mononucléose infectieuse, la maladie de Lyme, la poliomyélite, la salmonellose la fièvre typhoïde ou paratyphoïde, la tuberculose, le choléra, d'une infection causée par *Staphylococcus aureus*, une infection causée par pneumocoque, une infection causée par méningocoque, la fièvre jaune, les oreillons, Gastroentérite à rotavirus, la rougeole, la rubéole, la varicelle, le zona, l'hépatite, l'encéphalite japonaise, la coqueluche, la diphtérie, le tétanos, une infection causée par un coronavirus, une infection par un virus du papillome humain, une maladie à prions, une infection causée par un amibe, la syphilis, une mycose, le paludisme ou une infection causée par bactéries GNMR.

13. Composition immunogène ou vaccinale selon l'une quelconque des revendications précédentes, pour son utilisation dans la prévention et/ou le traitement d'une infection causée par au moins un agent pathogène chez l'humain ou chez un animal.

14. Composition immunogène ou vaccinale selon l'une quelconque des revendications précédentes, **caractérisée en ce qu'**elle comprend en outre une ou plusieurs substances additionnelles.

15. Composition immunogène ou vaccinale selon la revendication 14, **caractérisée en ce que** la substance additionnelle est choisi parmi :
- un conservateur,
- un tensioactif,
- un additif.
